# EUROPEAN PATENT SPECIFICATION

(11) **EP 2 922 567 B1**
(45) Date of publication and mention of the grant of the patent: **24.05.2017**
(21) Application number: 13794831.1
(22) Date of filing: 06.11.2013
(51) Int. Cl.: A61K 38/48, A61P 29/00

(54) **FIBRINOLYTIC COMPOSITIONS COMPRISING BROMELAIN AND NATTOKINASE FOR THE PREVENTION AND TREATMENT OF PHLEBOTHROMBOTIC STATES**
FIBRINOLYTISCHE ZUSAMMENSETZUNGEN DIE BROMELAIN UND NATTOKINASE ENTHALTEN ZUR PRÄVENTION UND BEHANDLUNG VON PHLEBOTHROMBOTISCHEN ZUSTÄNDE
COMPOSITIONS FIBRINOLYTIQUE COMPRENANT BROMELASE ET NATTOKINASE POUR LA PRÉVENTION ET LE TRAITEMENT D'ÉTATS PHLÉBOTHROMBOTIQUES

(30) Priority: 23.11.2012 IT MI20121997
(43) Date of publication of application: 30.09.2015
(73) Proprietor: Velleja Research SRL, 29010 Pontenure (PC) (IT); Gnosis S.p.A., 20121 Milano (MI) (IT)
(72) Inventor: DI PIERRO, Francesco, I-29010 Pontenure (PC) (IT)
(74) Representative: Bianchetti Bracco Minoja S.r.l.
(86) International application number: PCT/EP2013/073160
(87) International publication number: WO 2014/079689

(56) References cited:
- WO-A1-2009/070818
- WO-A2-2008/021987
- WO-A2-2011/063394
- MILNER M ET AL: "Natto and its active ingredient nattokinase: A potent and safe thrombolytic agent", ALTERNATIVE AND COMPLEMENTARY THERAPIES, MARY ANN LIEBERT, LARCHMONT, NY, US, vol. 8, no. 3, 1 June 2002 (2002-06-01), pages 157-164, XP009118951, ISSN: 1076-2809, DOI: 10.1089/107628002760091001
- "Bromelain. Monograph.", ALTERNATIVE MEDICINE REVIEW, vol. 15, no. 4, 1 December 2010 (2010-12-01), pages 361-368, XP055071521, ISSN: 1089-5159
- MAURER H R: "Bromelain: biochemistry, pharmacology and medical use", CMLS CELLULAR AND MOLECULAR LIFE SCIENCES, BIRKHAUSER VERLAG, HEIDELBERG, DE, vol. 58, no. 9, 1 August 2001 (2001-08-01) , pages 1234-1245, XP002995539, ISSN: 1420-682X, DOI: 10.1007/PL00000936
- CESARONE M R ET AL: "PREVENTION OF VENOUS THROMBOSIS IN LONG-HAUL FLIGHTS WITH FLITE TABS: THE LONFLIT-FLITE RANDOMIZED, CONTROLLED TRIAL", ANGIOLOGY, WESTMINSTER PUBLICATIONS, INC, GLEN HEAD, NY, US, vol. 54, no. 5, 1 January 2003 (2003-01-01), pages 531-539, XP009027252, ISSN: 0003-3197, DOI: 10.1177/000331970305400502
- TAI M -W ET AL: "Nattokinase for prevention of thrombosis", AMERICAN JOURNAL OF HEALTH-SYSTEM PHARMACY 20060615 US, vol. 63, no. 12, 15 June 2006 (2006-06-15) , pages 1121-1123, XP009171249, ISSN: 1079-2082

## Description

The present invention concerns fibrinolytic compositions for the prevention and treatment of phlebothrombotic states.

### Background of the invention

Phlebothrombosis is a disease characterised by the formation of thrombi in the lumen of one or more veins, with non-inflammatory causes, unlike phlebitis.

Several causes can induce the disease, and may occur both individually or variously combined: venous wall modifications, sluggish blood flow, or changes in blood coagulability. These phenomena are more frequent in individuals with a poor general state of health, diabetics, cardiopathic patients and post-surgical convalescent patients.

Most frequently, the lower limbs are affected, as well as the pelvic veins, the portal vein and the dural sinuses. The forming thrombi adhere weakly to the vessel wall, thus easily detaching and becoming emboli, which are able to occlude other distal vessels.

The thrombus can occlude the vessel, completely stopping the blood flow. The vessel is subsequently invaded by connective tissue, transforming the vessel into a fibrous mass. Small new blood vessels can form, allowing the total or partial re-establishment of the blood flow. Phlebothrombosis-related disorders, and the derived clinical pattern, differ according to the site and importance of the vessel involved; the larger the area, the more evident are the effects deriving from the impaired circulation: turgidity of the vascular venous stream-bed, oedema of the surrounding tissue area, a bluish colour, and more or less acute pain. If the vessel is small, phlebothrombosis may be asymptomatic and heal, while in other cases complications may arise, the most severe being pulmonary embolism.

The treatment of phlebothrombosis involves immobilisation of the limb. The use of thrombolytic drugs is also indicated.

Bromelain is a mixture of cysteine and protease extracted from the fruit and stem of the pineapple (*Ananas comosus),* belonging to the *Bromeliaceae* plant family. The molecular mass of the components ranges from 8 to 28.5 kDa.

Bromelain concentration is expressed in proteolytic units (GDU units or International Units, IU) or weight units. Bromelain is mainly used as an anti-inflammatory and anti-oedematous agent.

It is particularly efficacious in inflammatory states of the soft tissues, even following traumatic events and post-surgical tissue reactions. It has been demonstrated that the anti-inflammatory activity of bromelain is due to its ability to increase the biosynthesis of anti-inflammatory prostaglandins (i.e. prostaglandins E2) and inhibit pro-inflammatory prostaglandins.

In its anti-inflammatory use, bromelain is administered in 40 mg doses repeated two to six times per day, although much higher doses have been tested without any appreciable side effects, with the sole exception of slight gastrointestinal disorders and rare hypersensitivity reactions. Other pharmacological activities of bromelain include anti-thrombotic and pro-fibrinolytic actions, a hypotensive activity and the ability to solubilise atherosclerotic plaques. Synergic activities in antibiotic and antitumoral therapies have also been described. Bromelain is also used in dyspepsia, combined with the pancreatic juices.

Nattokinase is a proteolytic enzyme extracted from natto, a traditional Japanese food based on boiled soybean fermented by a particular *Bacillus subtilis*: *B. subtilis natto.*

Nattokinase is a serine-protease of about 30 kDa, belonging to the subtilisine family, with a strong fibrinolytic activity. For this reason it is employed as a substitute for other anti-aggregant and anti-coagulant drugs such as acetylsalicylic acid, ticlopidine and warfarin.

WO 200821987 discloses a preparation containing various enzymes (a fungal protease, papain, bromelain, fungal pancreatin, amylase, nattokinase, protease-S and lipase) combined with herbal derivatives (rutin and ginger) and trace elements (calcium, magnesium, potassium and other elements) for the prevention and treatment of thrombophlebitis.

The object of the present invention is a combination of only two enzymes, namely bromelain and nattokinase, in specific ratios and appropriately formulated, which present a particularly advantageous synergic effect.

### Description of the invention

The present invention relates to fibrinolytic compositions for the prevention and treatment of phlebothrombotic states, containing bromelain and nattokinase as the only active ingredients in the weight ratio of 1.6:1, and the combined use of bromelain and nattokinase in the same weight ratio in view of their fibrinolytic properties.

According to a preferred aspect, the compositions according to the invention will contain the active ingredients within the following ranges:
a) bromelain: between 20 and 400 mg, preferably 160 mg/dose/day; and
b) nattokinase: used in doses of between 10 and 100 mg.
   According to a more preferred aspect, the compositions according to the invention will contain the active ingredients at the following doses:
c) bromelain: 160 mg/dose/day; and
d) nattokinase: 100 mg/dose/day.

According to a preferred aspect, the compositions according to the invention will be formulated as bi-layer tablets, with one enzyme per layer.

### Clinical trial

The tests of the thrombolytic efficacy of preparations of bromelain and nattokinase were conducted according to the Astrupt model (The fibrin plate method for estimating fibrinolytic activity. Arch. Biochem. Biophys.; 40:346-351, 1952), performing each test in triplicate on bovine fibrin in the presence of 24 units of plasminogen, verifying the fibrinolytic action of bromelain and nattokinase individually, and mixing the two substances in various weight ratios. In the model described above, the action of bromelain was 11.5±2.5 FU/ml, while that of nattokinase was 18.9±3.3 FU/ml. When the two enzymes were mixed together in the ratio of 1:1, the fibrinolytic action was 97.8 ± 13.3 FU/ml, with a clear synergic effect, which was confirmed by reducing the doses of both enzymes by 1/10th and retesting the fibrinolytic action of the two compounds in a 1:1 mixture by weight. Operating in this way, the fibrinolytic action was 12.7 ± 2.9 FU/ml, a value similar to that obtainable by using both enzymes at doses 10 times higher.

When further tests were performed, it was observed that the best results were obtained by mixing bromelain and nattokinase in the weight ratio of 1.6:1.

The efficacy of the combination of bromelain and nattokinase was also evaluated by comparison with the combination of proteolytic enzymes and other ingredients described in WO 2008/02198, again using the Astrupt model. The fibrinolytic action of the individual compounds, the total mixture and the mixture of individual enzymes only, i.e. without the herbal derivatives, was therefore evaluated. We observed that individually, the majority of compounds (fungal protease, papain, pancreatin, amylase and protease-S) were far less active than bromelain and nattokinase. The latter two, which were active at values of 125±13.2 FU/ml and 139.5±34.7 FU/ml respectively, proved no less than 100 times more active than the other single enzymes. However, the herbal derivatives and trace elements proved totally inactive. The mixture of all ingredients (enzymes, herbal derivatives and trace elements) led to fibrinolytic activity, but less than that obtained by the mixture of bromelain and nattokinase alone. The fibrinolytic action of the total mixture was 47.9 ± 11.8 FU/ml, that of the mixture of all the enzymes 52.7 ± 8.4 FU/ml, and that of the combination of bromelain and nattokinase 1127.67 ± 119.6 FU/ml. These tests clearly demonstrate that the combination of bromelain and nattokinase is more effective than the combination claimed in WO200821987 and the combination of the single enzymes described therein. This phenomenon may be associated with possible antagonisms created by mixing different protein structures, and protein structures with molecules of herbal derivation, which compete for the same molecular epitopes on fibrin, making the fibrinolytic process inefficient or not very efficient.

### Formulations

The compositions according to the invention can be formulated in a way suitable for oral administration, and will be prepared by conventional methods well known in pharmaceutical technology, such as those described in "Remington's Pharmaceutical Handbook", Mack Publishing Co., N.Y., USA, using excipients, diluents, fillers, anti-caking agents, flavourings and sweeteners acceptable for their final use.

As mentioned, according to a preferred aspect, the compositions according to the invention will be formulated as bi-layer tablets, with one enzyme per layer; both layers will be gastroprotected, with time-dependent controlled intestinal release lasting about 4-8 hours. This type of release is made possible by the use, in the central nucleus of each layer, of methocel or metholose with different degrees of polymerisation, mixed in the ratio of 1:1 with avicel or microcel, which in turn are pre-mixed in the ratio of 1:1 with a mixture of mannitol and dicalcium phosphate, preconstituted in the ratio of 1:0.5. The gastroprotected film-coating is then obtainable using shellac directly in an industrial coating pan with a capacity of between 10 and 300 kg, preferably 50 kg. Finally, the film-coating can be made stronger by a further step in the industrial coating pan and the concomitant use of 1:0.5:0.5 mixtures of ammonium carbonate, triethyl citrate and shellac.

Formulation examples according to the invention are set out below.

### Example 1) Gastroprotected controlled-release tablets containing:

- bromelain 80 mg
- nattokinase 50 mg

### Example 2) Bi-layer gastroprotected controlled-release tablets containing, per layer:

- bromelain 160 mg
- nattokinase 100 mg

### Example 3) Gastroprotected granulate, obtained on fluid bed, containing:

- bromelain 320 mg
- nattokinase 200 mg

## Claims

1. Compositions comprising bromelain and nattokinase as the only active ingredients, wherein the weight ratio between bromelain and nattokinase is 1.6:1.

2. Compositions according to claim 1 in a form suitable for oral administration.

3. Compositions according to claim 2 in the form of bi-layer tablets, in which each layer contains a single enzyme, and is gastroprotected, with time-dependent controlled intestinal release lasting about 4-8 hours.

4. Combination of bromelain and nattokinase in the weight ratio of 1.6:1 for use in the treatment of thrombophlebitis.

## Patentansprüche

1. Zusammensetzungen, umfassend Bromelain und Nattokinase als die alleinigen aktiven Bestandteile, wobei das Gewichtsverhältnis zwischen Bromelain und Nattokinase 1,6:1 beträgt.

2. Zusammensetzungen gemäß Anspruch 1 in einer Form, die für eine orale Verabreichung geeignet ist.

3. Zusammensetzungen gemäß Anspruch 2 in der Form von Zweischichttabletten, worin jede Schicht ein einziges Enzym enthält und gegen Magensaft geschützt ist, mit einer zeitabhängigen gesteuerten intestinalen Freisetzung, die etwa 4-8 Stunden anhält.

4. Kombination von Bromelain und Nattokinase in dem Gewichtsverhältnis von 1,6:1 zur Verwendung bei der Behandlung von Thrombophlebitis.

## Revendications

1. Compositions comprenant de la bromélaïne et de la nattokinase comme seuls principes actifs, dans lesquelles le rapport en poids entre la bromélaïne et la nattokinase est de 1,6 : 1.

2. Compositions selon la revendication 1 sous une forme appropriée pour l'administration orale.

3. Compositions selon la revendication 2 sous forme de comprimés bi-couches, dans lesquelles chaque couche contient un seul enzyme, et est protégé gastriquement, avec une libération intestinale contrôlée dépendante du temps durant environ 4 à 8 heures.

4. Combinaison de bromélaïne et de nattokinase dans le rapport en poids de 1,6 : 1 pour une utilisation dans le traitement de la thrombophlébite.
